# EUROPEAN PATENT APPLICATION

(11) **EP 4 233 845 A1**
(43) Date of publication of application: **30.08.2023**
(21) Application number: 22461508.8
(22) Date of filing: 28.02.2022
(51) Int. Cl.: A61K 9/16, A61K 31/549

(54) **A PROCESS FOR MANUFACTURING HYDROCHLOROTHIAZIDE HAVING FINE PARTICLE SIZE DISTRIBUTION**

(71) Applicant: Zaklady Farmaceutyczne Polpharma S.A., 83-200 Starogard Gdanski (PL)
(72) Inventor: SAGOL, Karol, 83-110 Tczew (PL); KRZYZANOWSKI, Mariusz, 83-220 Skorcz (PL); WIELESIK, Cezary, 83-200 Starogard Gdanski (PL)

(57) **Abstract**

The present invention relates to a process for manufacturing hydrochlorothiazide having high purity and fine particle size distribution. Specifically, particle size distribution of hydrochlorothiazide manufactured in the process of the present invention is characterized by d10 ≤ 20 µm, d50 ≤ 50 µm and d90 ≤ 100 µm.

## Description

The present invention relates to a process for manufacturing hydrochlorothiazide having high purity and fine particle size distribution. Specifically, particle size distribution of hydrochlorothiazide manufactured in the process of the present invention is characterized by d10 ≤ 20 µm, d50 ≤ 50 µm and d90 ≤ 100 µm.

### Background art

Hydrochlorothiazide, i.e. 6-chloro-3,4-dihydro-2H-1,2,4-benzothiadiazine-7-sulfonamide 1,1-dioxide, has the following structural formula (1):

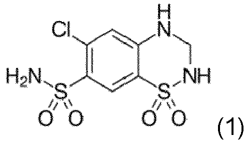

Hydrochlorothiazide is a diuretic drug that belongs to the thiazide class of diuretics and acts by inhibiting the kidney's ability to retain water. It is indicated alone or in combination for the management of hypertension, edema associated with congestive heart failure, hepatic cirrhosis, nephrotic syndrome, acute glomerulonephritis, chronic renal failure, and corticosteroid and estrogen therapy.

Hydrochlorothiazide is practically insoluble in water. It is classified as BCS class IV drug with poor aqueous solubility and low permeability leading to poor oral absorption. Active ingredients of that class show many problems in formulating them in dosage forms. There are number of formulation approaches to resolve the problems of low solubility and low bioavailability. Micronization is conventional and one of the most important means for enhancing drug solubility and thereby its oral bioavailability. Micronization reveals however some drawbacks. It may lead to at least partial amorphization of a solid what in turn negatively affects its flowability. Depending on properties of an active substance mechanical stress inherently accompanying micronization can also lead to chemical degradation of the active substance.

That last phenomenon has been observed for hydrochlorothiazide. Mechanical stress, in particular occurring during micronization, leads to formation of nitroso-derivative of hydrochlorothiazide, i.e. 6-chloro-4-nitroso-3,4-dihydro-2H-1,2,4-benzothiadiazide-7-sulfonamide-1,1-dioxide.

Without wishing to be bound by any theory, it is hypothesized that mechanical stress causes local increase of temperature which in turn promotes formation of 4-nitroso derivative of hydrochlorothiazide on the surface of its particles.

While carcinogenicity of nitroso-derivative of hydrochlorothiazide is unknown, there is a general presumption that N-nitroso-compounds are at least potential carcinogens and according to recommendations of CHMP of the European Medicines Agency ("Nitrosamine impurities in human medicinal products" EMEA/H/A-5(3)/1490), a control strategy regarding N-nitrosamines in active substances and finished products should be applied in view of potential formation of or contamination with N-nitrosamines.

This puts a significant burden upon API manufacturers who should find methods of avoiding or eliminating N-nitroso-impurities in their products and if possible, to minimise a risk that during manufacturing of the active ingredient the content of N-nitroso-impurities would increase to an unacceptable level.

WO2009/150497 A1 discloses a process for the preparation of hydrochlorothiazide which enables to reduce the content of dimer and trimer impurities in hydrochlorothiazide. The process involves reacting 5-chloro-2,4-disulfamyl aniline with paraformaldehyde in the absence of acid or base followed by purification by dissolving in aqueous solution of ammonia and sodium hydroxide and adjusting pH using hydrochloric acid. The present inventors have found that the final product of the purification as disclosed in that document has indeed high purity, but when trying to follow the procedure of purification as disclosed in Example 1 (b), the obtained hydrochlorothiazide had not a reproducible and not desired final granulometry.

There is therefore a need to develop a process which would allow to obtain high-purity hydrochlorothiazide of desired fine particle size distribution and which would not require micronization.

The process of the present invention solves the above problem.

### Disclosure of the invention

In the present invention, hydrochlorothiazide particles are characterized by their particle size distribution (PSD), represented by its d90, d50 and/or d10 value.

The d90 value is defined such that 90 % by volume of the particles have a particle size smaller than the d90 value and 10 % by volume of the particles have a greater particle size greater than the d90 value. The d50 value and the d10 value are defined accordingly.

PSD in the present disclosure is determined by volume using laser-diffraction wet method.

The term "crude hydrochlorothiazide" as used therein, means hydrochlorothiazide requiring particle size reduction to be used in solid pharmaceutical compositions. In particular, crude hydrochlorothiazide is characterized by d10 > 20 µm, d50 > 50 µm and d90 > 100 µm. Although the process according to the present invention can lead to certain improvement in chemical purity, the crude hydrochlorothiazide in the context of the present invention can already have chemical purity which is acceptable for use in pharmaceuticals.

In the first aspect, the present invention relates to a process for manufacturing of hydrochlorothiazide having particle size distribution d10 ≤ 20 µm, d50 ≤ 50 µm and d90 ≤ 100 µm comprising:
(a) dissolving crude hydrochlorothiazide in aqueous solution comprising one or more alkali metal hydroxide(s) and/or one or more alkaline earth metal hydroxide(s), and ammonia and/or one or more amine(s),
(b) adding an aqueous solution of an organic or an inorganic acid to the solution of step (a) to adjust pH value of the solution of step (a) to 6 - 8, preferably to 6,5 - 7,5, while maintaining temperature within a range of 15 - 35°C, and then optionally diluting the mixture with water,
(c) heating the mixture of step (b) to 30 - 45°C and maintaining it at that temperature for at least 1 hour,
(d) isolating hydrochlorothiazide having particle size distribution characterized by d10 ≤ 20 µm, d50 ≤ 50 µm and d90 ≤ 100 µm.

In the preferred embodiment step (a) comprises the following sub-steps:
(a1) adding crude hydrochlorothiazide to aqueous solution of one or more alkali metal hydroxide(s) and/or one or more alkaline earth metal hydroxide(s), and
(a2) adding aqueous ammonia and/or aqueous solution of the one or more amine to solution of step (a1) and stirring the mixture at room temperature to obtain a solution.

Preferably, in the process according to the present invention, in step (a) or step (a1) a molar ratio of crude hydrochlorothiazide to one or more alkali metal hydroxide(s) and/or one or more alkaline earth metal hydroxide(s) is within the range of from 1:1,5 to 1:2,5, preferably it is 1:1,9.

Further preferably, in the process according to the present invention, in step (a) or step (a2) a molar ratio of crude hydrochlorothiazide to ammonia and/or the one or more amine is within the range of from 1:0,6 to 1:1, preferably it is 1:0,8.

According to another preferred embodiment of the process of the present invention, the one or more alkali metal hydroxide(s) and/or one or more alkaline earth metal hydroxide(s) used in step (a) is one or more alkali metal hydroxide(s). More preferably, in the process according to the present invention one or more alkali metal hydroxide(s) is selected from lithium hydroxide, potassium hydroxide, sodium hydroxide and a mixture thereof, and most preferably it is sodium hydroxide.

In another preferred embodiment of the process according to the present invention, a concentration of one or more alkali metal hydroxide(s) and/or one or more alkaline earth metal hydroxide(s), in the aqueous solution of step (a1) is within the range of 3-10% w/w, preferably it is 5% w/w, with regard to the total weight of the aqueous solution of step (a1).

Further preferably, in the process of the present invention, ammonia is used in step (a) or step (a2).

In the preferred embodiment, 25% w/w aqueous ammonia is used in step (a2).

Preferably, dissolving hydrochlorothiazide in step (a) or (a1)+(a2) is carried out at a temperature of 20-25°C.

In one preferred embodiment of the process according to the present invention, the acid used in step (b) is an inorganic acid, preferably a strong mineral acid. In the most preferred embodiment, the acid used in step (b) is hydrochloric acid, preferably the concentrated hydrochloric acid (37% w/w).

Addition of acid in step (b) results in neutralisation of solution of step (a) ((a1)+(a2)) wherein hydrochlorothiazide precipitates.

Maintaining temperature within a range of 15 - 35°C in step (b) and within a range of 30 - 45°C, preferably 38 - 42°C, in heating step (c), appeared to be crucial parameters to ensure the desired particle size distribution of the final hydrochlorothiazide.

Controlling the temperature regime as indicated above can be performed by means conventionally used in the art. It will be apparent fort he skilled person that efficient agitation and heating/cooling equipment, i.e. a reactor with a heating/cooling mantle ensuring efficient heat transfer would be necessary to perform the process of the present invention.

Preferably, time of maintenance in step (c) ranges within 1-3 hours, more preferably 1-2 hours.

In one preferred embodiment of the process of the present invention, hydrochlorothiazide as isolated in step (d) has particle size distribution characterized by d10 within a range of 2-10 µm, d50 within a range of 5-50 µm and d90 within a range of 20-100 µm. More preferably, the process according tot he present invention allows to obtain hydrochlorothiazide having particle size distribution characterized by d10 within a range of 2-10 µm, d50 within a range of 10-30 µm and d90 within a range of 40-80 µm.

Hydrochlorothiazide can be isolated by conventional means, i.e. using procedures and equipment conventionally used in the art. In particular, hydrochlorothiazide precipitate of step (c) can be filtered off using a centrifuge, washed with water, then subject to drying in a mixer-dryer or in a tray dryer at a temperature of from 80 to 120°C. Preferably, drying

is carried out at a temperature of from 90 to 100°C.

In a second aspect, the present invention relates to a pharmaceutical composition containing hydrochlorothiazide, as obtained according to the process of the first aspect of the present invention, in combination with one or more pharmaceutically acceptable excipient(s).

In the preferred embodiment of the above second aspect, the pharmaceutical composition is formulated into a solid unit dosage form for oral administration, preferably into a tablet.

Pharmaceutically acceptable excipients which can be contemplated for use in the pharmaceutical composition of the present invention are conventional and known to the skilled person. For example, pharmaceutically acceptable excipients are described in P.J. Sheskey (Ed.), Handbook of Pharmaceutical Excipients: Edition 9, Pharmaceutical Press, 2020.

The present invention will now be described with reference to the following examples, which are not intended to be limiting.

### EXAMPLES

### HPLC method: European Pharmacopoeia 10.0, Monograph for Hydrochlorothiazide

| Equipment | HPLC apparatus equipped with spectrophotometer detector | | |
|---|---|---|---|
| Column | Hypersil ODS C18, I = 0,1 m, Ø = 4,6 mm | | |
| Mobile phase A | Phosphate buffer pH 3,2 940 ml, methanol 60,0 ml, THF 10 ml | | |
| Mobile phase B | Methanol 500 ml, phosphate buffer pH 3,2 500 ml, THF 50 ml | | |
| Gradient | Time | Mobile phase A | Mobile phase B |
| | [min] | [% v/v] | [% v/v] |
| | 0-17 | 100 -> 55 | 0 -> 45 |
| | 17-30 | 55 | 45 |
| | 30-35 | 55 -> 100 | 45 -> 0 |
| | 35-50 | 100 | 0 |
| Acquisition time | 10.0 minutes | | |
| Mobile phase flow | 0.8 ml/min | | |
| Column temperature | 25°C | | |
| Autosampler temperature | 15°C | | |
| Injection volume | 10.0 µl | | |

### PSD studies:

| System | Malvern 2000 ver. 5.40 |
|---|---|
| Dispersion Unit | Hydro 2000 S |
| Sample dispersion way | Wet way |
| Liquid medium | Saturated hydrochlorothiazide solution |
| Measurement time | 10 s |
| Laser concentration | 10 - 25% |
| Pump speed | 2500 rpm |
| Optical model | Fraunhofer |

### Preparation of saturated hydrochlorothiazide solution

10 g of hydrochlorothiazide is added to 1000 ml of purified water; the mixture is placed on ultrasonic bath for 30 min and cooled to room temperature. The solution is filtered through a 0,2 um filter prior use. 50 ml of saturated hydrochlorothiazide solution is placed in 100 ml-beaker. 50 mg of hydrochlorothiazide to be analyzed is added thereto and the whole is stirred for 3 min to obtain a homogenous suspension.

### Analytical procedure

Measurement is performed according to the operating instructions (see parameters in the table above). Two successive determinations are performed, from which the mean value is calculated.

### Example 1 - Preparation of crude hydrochlorothiazide

To a mixture of 35,8 g of sulfuric acid in 20 I of water, 500,6 g of 4-amino-6-chlorobenzene-1,3-disulphonamide acetamide was added followed by adding 59,2 g of paraformaldehyde. The mixture was heated and refluxed for 2 hours and then cooled to room temperature within 3 hours. Product was filtered off, washed with 6000 ml of water and dried under vacuum at 100°C to obtain crude hydrochlorothiazide.
Yield: 500,0 g (88 %)
Purity (HPLC): 99,71%
PSD: d10: 70 µm, d50: 150 µm, d90: 300 µm

### Example 2.

25 g of Hydrochlorothiazide of Example 1 were added to a solution of 8,18 g of NaOH in 175 ml of water at room temperature when stirred. Then 5 ml of 25% aqueous solution of ammonia were added . The solution was stirred for 1 hour, followed by adjusting pH value to 6,5-7 using concentrated hydrochloric acid (37% w/w in water). Temperature during acid addition was kept at 25-30°C using cooling bath. The cooling bath was removed and to the resulting reaction mass 125 ml of water (room temperature) was added. The whole was heated and stirred for additional hour at about 40°C. The solid product was filtered off, washed with 480 ml of water and dried under vacuum at 100°C.
Yield: 22,8 g (91,2%)
Purity (HPLC): 99,88%
PSD: d10: 9,6 µm, d50: 28 µm, d90: 65 µm

### Example 3.

25 g of Hydrochlorothiazide of Example 1 were added to a solution of 6,5 g of NaOH in 124 ml of water at room temperature when stirred. Then 5 ml of 25% aqueous solution of ammonia were added. The solution was stirred for 1 hour, followed by adjusting pH value to 6,5-7 using concentrated hydrochloric acid (37% w/w in water). Temperature during acid addition was kept at 20-22°C using cooling bath. The cooling bath was removed and to the resulting reaction mass 125 ml of water (room temperature) was added. The whole was heated and stirred for additional hour at about 40°C. The solid product was filtered off, washed with 480 ml of water and dried under vacuum at 100°C.
Yield: 22,3 g (89,2%)
Purity (HPLC): 99,92%
PSD: d10: 7,2 µm, d50: 23 µm, d90: 51 µm

## Claims

1. A process for manufacturing of hydrochlorothiazide having particle size distribution of d10 ≤ 20 µm, d50 ≤ 50 µm and d90 ≤ 100 µm comprising:
(a) dissolving crude hydrochlorothiazide in aqueous solution comprising one or more alkali metal hydroxide(s) and/or one or more alkaline earth metal hydroxide(s), and ammonia and/or one or more amine(s),
(b) adding an aqueous solution of an organic or an inorganic acid to the solution of step (a) to adjust pH value of the solution of step (a) to 6 - 8, preferably to 6,5 - 7,5, while maintaining temperature within a range of 15 - 35°C, and then optionally diluting the mixture with water,
(c) heating the mixture of step (b) to 30 - 45°C and maintaining it at that temperature for at least 1 hour,
(d) isolating hydrochlorothiazide having particle size distribution **characterized by** d10 ≤ 20 µm, d50 ≤ 50 µm and d90 ≤ 100 µm.

2. The process according to claim 1, wherein step (a) comprises:
(a1) adding crude hydrochlorothiazide to aqueous solution of one or more alkali metal hydroxide(s) and/or one or more alkaline earth metal hydroxide(s), and
(a2) adding aqueous ammonia and/or aqueous solution of the one or more amine to a mixture of step (a1) and stirring the mixture at room temperature to obtain a solution.

3. The process according to claims 1 or 2, wherein molar ratio of crude hydrochlorothiazide to one or more alkali metal hydroxide(s) and/or one or more alkaline earth metal hydroxide(s) is within the range of 1:1,5 to 1:2,5, preferably it is 1:1,9.

4. The process according to any one of claims 1 to 3, wherein molar ratio of crude hydrochlorothiazide to ammonia and/or the one or more amine is within the range of 1:0,6 to 1:1.preferably it is 1:0,8.

5. The process according to any one of claims 1 to 4, wherein one or more alkali metal hydroxide(s) and/or one or more alkaline earth metal hydroxide(s) used in step (a) is one or more alkali metal hydroxide(s).

6. The process according to claim 5, wherein one or more alkali metal hydroxide(s) is selected from lithium hydroxide, potassium hydroxide, sodium hydroxide and a mixture thereof, preferably it is sodium hydroxide.

7. The process according to any one of claims 2 to 6, wherein concentration of one or more alkali metal hydroxide(s) and/or one or more alkaline earth metal hydroxide(s), in the aqueous solution of step (a1) is within the range of 3-10% w/w, preferably it is 5% w/w, with regard to the total weight of the aqueous solution of step (a1).

8. The process according to any one of claims 1 to 7, wherein ammonia and/or one or more amine used in step (a) is ammonia.

9. The process according to any of the claims 2 to 8, wherein 25% w/w aqueous ammonia is used in step (a2).

10. The process according to any one of claims 1 to 9, wherein the acid used in step (b) is an inorganic acid, preferably a strong mineral acid.

11. The process according to claim 10, wherein the acid used in step (b) is hydrochloric acid, preferably the concentrated hydrochloric acid (about 37% w/w).

12. The process according to any of the claims 1-11, wherein maintenance time in step (c) is within a range of 1-3 hours, preferably 1-2 hours.

13. The process according to any of the claims 1-12, wherein, hydrochlorothiazide isolated in step (d) has particle size distribution **characterized by** d10 within a range of 2-10 µm, d50 within a range of 5-50 µm and d90 within a range of 20-100 µm, more preferably, **characterized by** d10 within a range of 2-10 µm, d50 within a range of 10-30 µm and d90 within a range of 40-80 µm.

14. The process according to claim 1, comprising:
(a1) adding crude hydrochlorothiazide to aqueous solution of sodium hydroxide,
(a2) adding aqueous ammonia to a mixture of step (a1) and stirring the mixture at room temperature to obtain a solution,
(b) adding an aqueous solution of hydrochloric acid to the solution of step (a) to adjust pH of the mixture to 6 - 8, preferably 6,5-7,5, while maintaining temperature within a range of 15 - 35°C, and then optionally diluting the mixture with water,
(c) heating the mixture of step (b) to 30 - 45°C, preferably to 38 - 42°C, and maintaining it at that temperature for at least 1 hour,
(d) isolating hydrochlorothiazide having particle size distribution **characterized by** d10 within a range of 2-10 µm, d50 within a range of 5-50 µm and d90 within a range of 20-100 µm, more preferably, **characterized by** d10 within a range of 2-10 µm, d50 within a range of 10-30 µm and d90 within a range of 40-80 µm.
